# EUROPÄISCHE PATENTSCHRIFT

(11) **EP 0 546 412 B1**
(45) Veröffentlichungstag und Bekanntmachung des Hinweises auf die Patenterteilung: **06.05.1999**
(21) Anmeldenummer: 92120414.5
(22) Anmeldetag: 30.11.1992
(51) Int. Cl.: G01N 33/42, G01N 33/28, G01N 27/00, G01N 31/00

(54) **Verfahren und Einrichtung zur Prüfung der Auslaugbeständigkeit eines Materials**
Method and apparatus for testing leach resistance of a material
Méthode et appareil pour tester la résistance au lessivage d'un matériel

(30) Priorität: 13.12.1991 DE 4141231
(43) Veröffentlichungstag der Anmeldung: 16.06.1993
(73) Patentinhaber: SIEMENS AKTIENGESELLSCHAFT, 80333 München (DE)
(72) Erfinder: Ahrens-Botzong, Rudolf, Dr., W-6050 Offenbach (DE)

(56) Entgegenhaltungen:
- EP-A- 0 302 310
- DE-A- 4 021 103
- US-A- 4 375 451
- US-A- 4 737 356
- US-A- 5 033 397
- ENTSORGUNGS PRAXIS, Nr. 6, Juni 1990 Seiten 367-372, J.FRIGGE 'Prüfung des Deponierverhaltens reaktiver Massengüter mit praxisnahen Grosslysimetern'
- PATENT ABSTRACTS OF JAPAN vol. 005 no. 038 (P-052) ,12.März 1981 & JP-A-55 160846 (NIKKO TANKAI KK) 15.Dezember 1980,

## Beschreibung

Die Erfindung betrifft ein Verfahren zur Prüfung eines Materials, insbesondere von Schmelzgranulat aus einer Abfallbehandlungsanlage, wobei das Material in eine Flüssigkeit eingebracht und seine Beständigkeit gegen Auslaugung geprüft wird.

Die Erfindung betrifft auch eine Einrichtung zur Prüfung eines Materials auf Auslaugbeständigkeit, insbesondere von Schmelzgranulat aus einer Abfallbehandlungsanlage, mit einem Behälter zur Aufnahme einer Flüssigkeit und des Materials.

Bei der thermischen Abfallbehandlung und auch in fossil befeuerten Kraftwerken fallen Reststoffe an, die weiter verwendet werden können. Solche Reststoffe sind beispielsweise als Baustoffe, insbesondere für den Straßenbau, tauglich. Ein solcher Reststoff ist auch das Schmelzgranulat, das beim aus der europäischen Patentschrift 0 302 310 bekannten Schwel- Brenn-Verfahren anfällt.

Damit ein Reststoff als Baustoff oder als anderer Wertstoff eingesetzt werden darf, muß sichergestellt sein, daß er an seinem Einsatzort keine Schadstoffe freisetzt. Es soll daher sichergestellt werden, daß der Kontakt mit Regenwasser oder auch mit anderem Wasser nicht zu einer Auslaugung des Materials und damit zu einem Übergang von Schadstoffen in das Wasser führt. Ein Reststoff sollte nur dann weiterverwendet werden, wenn eine Berührung mit Wasser, das wechselnde Salzkonzentrationen, pH-Werte oder Redoxpotentiale hat, nicht zu einem Auslaugen führt. Selbst die Einwirkung von Komplexbildnern soll kein Auslaugen zur Folge haben. Die Auslaugbeständigkeit eines Stoffes ist eine Bewertungsgröße, die auf ein Wertesystem zurückzuführen ist. Zu diesem Wertesystem können z.B. die Löslichkeit in Flüssigkeiten oder mechanische Stabilität gehören.

Um festzustellen, ob ein Reststoff weiterverwendet werden darf, ist es üblich, Auslaugtests vorzunehmen. Dabei wird das Material, das auf seine Auslaugbeständigkeit geprüft werden soll, im Labor denjenigen Bedingungen ausgesetzt, die beim Einsatz als Wertstoff zu erwarten sind. Das Material wird dazu beispielsweise in eine Flüssigkeit eingebracht. Falls danach im Laufe der Zeit eine Schadstoffkonzentration in der Flüssigkeit meßbar wird oder diese ansteigt, wird darauf geschlossen, daß die Schadstoffe aus dem zu prüfenden Material ausgelaugt worden sind. Das Material ist folglich nicht auslaugbeständig.

Solche Verfahren sind beispielsweise aus dem Aufsatz von Dr. Joachim Frigge "Prüfung des Deponieverhaltens reaktiver Massengüter mit praxisnahen Großlysimetern", EntsorgungsPraxis 6/90, Seiten 367 bis 372, bekannt. Ein bekanntes Prüfverfahren ist der Schütteltest. Dabei wird das zu prüfende Material in ein Deionatvolumen eingebracht. Das Gefäß, in dem sich das Material und das Deionat befinden, wird geschüttelt. Das Verfahren wird mindestens 24 Stunden lang durchgeführt. Erst danach kann angenommen werden, daß das Material weitgehend ausgelaugt ist. Es können auch Auslaugzeiten von mehreren Tagen erforderlich sein. Nach dem Auslaugen erfolgt dann eine Filtration und eine Analyse des Filtrates. Aus den Filtrateigenschaften werden Rückschlüsse auf die Auslaugbeständigkeit des Materials gezogen. Falls Schadstoffe im Filtrat nachweisbar sind, ist das Material nicht auslaugbeständig.

Bekannte Prüfverfahren benötigen für die Prüfung einer Materialprobe einen oder sogar mehrere Tage. Damit soll sichergestellt sein, daß bei der Prüfung der Auslaugbeständigkeit im Labor möglichst ähnliche Bedingungen herrschen wie beim späteren Einsatz des Materials, wenn es als Baumaterial Niederschlägen oder anderen Wässern ausgesetzt ist.

Bekannte Prüfverfahren sind nicht geeignet für eine lückenlose Uberprüfung von Material, das laufend nachgeliefert wird. Wegen der langen Prüfzeit kann nur in großen Abständen eine Materialprobe auf Auslaugbeständigkeit überprüft werden. Sollte eine Probe den Anforderungen nicht entsprechen, müßte das gesamte Material, das seit der letzten zufriedenstellenden Prüfung angefallen ist, verworfen werden. Beim Einsatz bekannter Prüfverfahren besteht also die nicht geringe Wahrscheinlichkeit, daß große Mengen für eine Weiterverwendung ungeeigneten Materials von einer Anlage abgegeben werden, bevor durch das Prüfverfahren erkannt wird, daß dieses Material den Anforderungen nicht genügt. Das den Anforderungen nicht genügende Material muß dann einer Sonderbehandlung oder sogar einer Problemstoffdeponie zugeführt werden. Darüber hinaus würde dieses Material als Baumaterial nicht zur Verfügung stehen. Sollte ein fester Liefervertrag für Baumaterial bestehen, könnte dieser gegebenenfalls nicht eingehalten werden.

Der Erfindung liegt die Aufgabe zugrunde, ein Verfahren zur Prüfung der Auslaugbeständigkeit eines Materials, insbesondere von Schmelzgranulat aus einer Abfallbehandlungsanlage, anzugeben, das in kurzer Zeit ein zuverlässiges Ergebnis liefert. Die Prüfung soll so schnell erfolgen können, daß an einer Anlage, die laufend Material abgibt, nach Erkennen fehlender Auslaugbeständigkeit durch sofortige Maßnahmen verhindert werden kann, daß größere Mengen nicht auslaugbeständigen Materials anfallen.

Außerdem liegt der Erfindung die Aufgabe zugrunde, eine Einrichtung zur Prüfung der Auslaugbeständigkeit eines Materials anzugeben, die zur Durchführung des Verfahrens geeignet ist.

Die erstgenannte Aufgabe wird gemäß der Erfindung dadurch gelöst, daß in der Flüssigkeit nach dem Einbringen des Materials mindestens eine Zustandsgröße stetig, sprunghaft oder periodisch geändert und dabei der Verlauf mindestens einer Zustandsgröße als Prüfverlauf registriert wird, daß zuvor in einer gleichen Flüssigkeit ohne Materialeinbringung und sonst unter gleichen Bedingungen in gleicher Weise die gleiche mindestens eine Zustandsgröße geändert wird und der Verlauf der für den Prüfverlauf vorgesehenen Zustandsgröße als Kalibrierverlauf registriert wird und daß die Differenz von Prüfverlauf und Kalibrierverlauf gebildet wird, wobei die Differenz ein Maß für die Beständigkeit des Materials gegen Auslaugung ist.

Dabei können die geänderte und die registrierte Zustandsgröße die gleiche oder verschiedene Zustandsgrößen sein.

Durch die Änderung mindestens einer Zustandsgröße und das gleichzeitige Bestimmen der Änderung mindestens einer Zustandsgröße erhält man vorteilhafterweise einen oder mehrere Prüfverläufe, die sich schon bei geringfügiger Änderung der Auslaugbeständigkeit des Materials meßbar ändern. Um Einflüsse des Behältermaterials und andere äußere Einflüsse auszuschließen, werden ein oder mehrere Kalibrierverläufe ohne zu prüfendes Material bestimmt. Es wird dann später nur die Differenz zwischen einem gemessenen Prüfverlauf und einem dazu gehörenden Kalibrierverlauf betrachtet, um den Einfluß des Materials zu erkennen.

Mit dem Verfahren gemäß der Erfindung steht ein sehr empfindliches Prüfverfahren zur Verfügung. Wegen der hohen Empfindlichkeit kann die erforderliche Zeit für eine Prüfung so gering sein, daß sehr schnell nacheinander verschiedene Proben eines Materials auf Auslaugbeständigkeit geprüft werden können. Erst dadurch ist es vorteilhafterweise möglich, laufend anfallendes Material, wie beispielsweise Granulat einer Abfallbehandlungsanlage, so schnell zu prüfen, daß fast eine kontinuierliche Prüfung gegeben ist. Damit wird der Vorteil erzielt, daß beim Erkennen einer nicht ausreichenden Auslaugbeständigkeit die Gesamtanlage korrigiert oder stillgelegt werden kann, bevor große Mengen nicht auslaugbeständigen Materials anfallen können.

Es fällt also vorteilhafterweise kaum Material an, das nicht weiterverwendet werden könnte.

Die bestimmte Differenz von Prüfverlauf und Kalibrierverlauf ist Null, wenn während des Prüfverfahrens von dem zu überprüfenden Material nichts gelöst wird. Das Vorhandensein einer Differenz weist darauf hin, daß ein Teil des Materials in der Flüssigkeit gelöst wurde. Das Material ist folglich nicht auslaugbeständig.

Dadurch, daß die Zustandsgröße stetig, sprunghaft oder periodisch geändert wird, ergibt sich eine leicht zu erkennende Änderung derjenigen Zustandsgröße, die registriert wird. Das ist besonders bei einer sprunghaften Änderung gegeben.

Beispielsweise wird mindestens eine Zustandsgröße aus der Gruppe pH-Wert, Redoxpotential, elektrische Leitfähigkeit und Konzentration eines gelösten Stoffes geändert und dabei der Verlauf einer Zustandsgröße aus dieser Gruppe registriert. Bei einer Änderung mindestens einer Zustandsgröße aus dieser Gruppe ist zu erwarten, daß aus einem nicht auslaugbeständigen Material ein Teil des Materials gelöst wird. Außerdem können diese Zustandsgrößen mit bekannten Vorrichtungen gemessen werden.

Zur Änderung einer Zustandsgröße werden in die Lösung z.B. ein oder mehrere Reagenzien dosiert. Zur Änderung des pH-Wertes eignen sich saure oder alkalische Stoffe. Zur Änderung des Redoxpotentials eignen sich Oxidations- oder Reduktionsmittel. Die elektrische Leitfähigkeit kann durch Zugabe eines Salzes geändert werden.

Die Änderung der Zustandsgröße kann automatisch gesteuert werden. Dazu dient eine Dosiervorrichtung. Zur Erzeugung einer periodischen Änderung der Zustandsgröße müssen in periodischem Abstand entgegengerichtete Änderungen vorgenommen werden. Für eine periodische pH-Wertänderung müssen z.B. in periodischen Abständen saure und alkalische Mittel in die Flüssigkeit eingespeist werden. Eine solche Dosierung bedarf in der Regel einer automatischen Steuerung.

Beispielsweise wird das zu prüfende Material vorzerkleinert in die Flüssigkeit eingebracht. Dadurch hat das Material relativ zu seinem Volumen eine große Oberfläche, die mit der Flüssigkeit in Kontakt tritt. Es kann dann, falls eine Auslaugbeständigkeit nicht gegeben ist, ein Teil des Materials schnell gelöst werden. Daraus folgt, daß eine zu geringe Auslaugbeständigkeit schneller als bei unzerkleinertem Material erkannt werden kann.

Das Material wird beispielsweise von der Flüssigkeit umspült. Dadurch wird eine Änderung einer Zustandsgröße in der Flüssigkeit schnell auf die gesamte Flüssigkeit verteilt und folglich schneller registriert.

Die Flüssigkeit läuft beispielsweise in einem geschlossenen Kreislauf um. Dadurch wird das Material ausreichend umspült.

Die Flüssigkeit kann in dem geschlossenen Kreislauf unter einem gegenüber dem Außendruck erhöhten Druck umlaufen. Sie nimmt daher lösbares Material besser auf.

Außerdem kann die Temperatur der Flüssigkeit im geschlossenen Kreislauf gegenüber der Außentemperatur erhöht oder erniedrigt werden, um das Lösungsverhalten zu verändern.

Ein Gasraum oberhalb der Flüssigkeit kann mit einem Schutzgas gefüllt sein, um unerwünschte Reaktionen auszuschließen.

Der Verlauf einer Zustandsgröße kann in einem abgezweigten Teilstrom der Flüssigkeit registriert werden. Dann muß nicht die gesamte Flüssigkeit durch ein Meßgerät hindurchgeführt werden. Die Registrierung des Verlaufs kann aber auch in einem Hauptflüssigkeitskreislauf oder in ruhender Flüssigkeit erfolgen.

Der Verlauf kann außerdem hinter einem Filter registriert werden, um ein Meßgerät für eine Zustandsgröße vor Feststoffpartikeln zu schützen.

Beispielsweise kann während des Prüfverfahrens ein Teil der Flüssigkeit entfernt und verworfen werden. Das kann notwendig sein, wenn im Laufe des Verfahrens die Flüssigkeitsmenge durch die Zugabe von Reagenzien zur Änderung der Zustandsgröße zu stark angewachsen ist. Der Ablaßstrom kann automatisch gesteuert werden.

Mit dem Verfahren nach der Erfindung wird der Vorteil erzielt, daß eine Änderung der Auslaugbeständigkeit eines Materials so schnell registriert werden kann, daß eine fortlaufende Uberwachung eines Materialstromes möglich ist.

Die als zweite genannte Aufgabe, eine Einrichtung zur Prüfung eines Materials auf Auslaugbeständigkeit anzugeben, wird gemäß der Erfindung dadurch gelöst, daß der Behälter eine Öffnung aufweist zum Hineingeben eines Reagenz, durch das eine Zustandsgröße der Flüssigkeit geändert wird, daß dem Behälter mindestens ein Meßgerät zugeordnet ist zum Messen und Registrieren eines Prüfverlaufs einer Zustandsgröße und daß das mindestens eine Meßgerät mit einer Auswerteeinheit zum Bilden der Differenz aus dem Prüfverlauf und einem Kalibrierverlauf verbunden ist.

Dabei können die geänderte und die registrierte Zustandsgröße die gleichen oder verschiedenen Zustandsgrößen sein.

Mit der Einrichtung gemäß der Erfindung kann in kurzer Zeit festgestellt werden, ob das Material auslaugbeständig ist. Das kann so schnell erfolgen, daß ein fortlaufend anfallendes Material fast kontinuierlich in kurzen zeitlichen Abständen überprüft werden kann. Zwischen zwei aufeinander folgenden Prüfungen fällt daher nur wenig Material an. Wenn eine Prüfung ergibt, daß die Auslaugbeständigkeit nicht gegeben ist, liegt nur wenig Material vor, das seit der vorangegangenen Prüfung angefallen ist und das folglich nicht auslaugbeständig sein könnte. Eine fehlende Auslaugbeständigkeit wird schnell erkannt, wodurch die Bildung von nicht auslaugbeständigem Material kurzfristig unterbunden werden kann.

Der Öffnung im Behälter, die zum Hineingeben eines Reagenz dient, kann mindestens eine Dosiervorrichtung für ein Reagenz vorgeschaltet sein. Mit einer solchen Dosiervorrichtung kann eine Steuereinheit verbunden sein. Mit diesen Vorrichtungen kann eine Zustandsgröße in der Flüssigkeit stetig, sprunghaft oder periodisch geändert werden.

Dem Behälter ist beispielsweise eine Zerkleinerungsvorrichtung für das Material vorgeschaltet. Bei einem Material mit relativ großer Oberfläche ist die Prüfung auf Auslaugbeständigkeit mit erhöhter Zuverlässigkeit durchführbar. Manches Material zeigt nämlich, wenn es als relativ großvolumiger Block vorliegt, eine relativ große Auslaugbeständigkeit, die es sonst nicht hat.

Der Behälter ist z.B. in einem Flüssigkeitskreislauf, in dem sich eine Pumpe befindet, angeordnet. Die Flüssigkeit wird dadurch ständig bewegt. Möglicherweise aufgelöstes Material wird dadurch in der gesamten Flüssigkeit verteilt und kann über eine Veränderung einer Zustandsgröße schnell erkannt werden.

Ein oder mehrere Meßgeräte für eine oder mehrere Zustandsgrößen sind z.B. im Flüssigkeitskreislauf außerhalb des Behälters angeordnet. Dabei ist sichergestellt, daß die gesamte Flüssigkeit im Kreislauf das Meßgerät durchströmt, so daß eine gute Meßgenauigkeit erzielt wird.

Meßgeräte können aber auch im Behälter angeordnet sein.

Beispielsweise können Meßgeräte in einem Bypaß des Flüssigkeitskreislaufes angeordnet sein. Dann braucht bei ausreichender Durchmischung der Flüssigkeit ein Meßgerät nicht für den gesamten großen Flüssigkeitsstrom ausgelegt zu sein.

Einem Meßgerät kann ein Filter vorgeschaltet sein, um feste Partikel, die das Meßgerät stören könnten, zurückzuhalten.

Beispielsweise weist der Behälter einen Auslaß für überschüssige Flüssigkeit auf. Insbesondere in einem geschlossenen Kreislauf kann durch Zugabe von Reagenzien die Flüssigkeitsmenge zu groß werden. Dann muß ein Teil der Flüssigkeit abgelassen werden. Dieser Flüssigkeitsteil wird verworfen. Eine Armatur im Auslaß kann über die Steuereinheit steuerbar sein.

Der Behälter ist beispielsweise bis auf Anschlüsse geschlossen. Es kann daher im Behälter ein erhöhter Druck eingestellt werden, wodurch Material leichter gelöst wird und damit eine Uberprüfung der Auslaugbeständigkeit genauer erfolgen kann.

Im System kann beispielsweise eine geregelte Heizung oder Kühlung installiert sein, um die Flüssigkeit und das Material auf einer gewünschten Temperatur zu halten.

Die Einrichtung gemäß der Erfindung macht wie das Verfahren eine in kurzen Abständen erfolgende, fast kontinuierliche Prüfung eines Materials auf Auslaugbeständigkeit möglich. Da laufend gebildetes Material mit der gleichen Geschwindigkeit, mit der es gebildet wird, überprüft werden kann, kann auf eine festgestellte zu kleine Auslaugbeständigkeit sofort reagiert werden. Es kann verhindert werden, daß eine große Menge nicht auslaugbeständiges Material anfällt, bevor erkannt wird, daß dieses Material nicht auslaugbeständig ist.

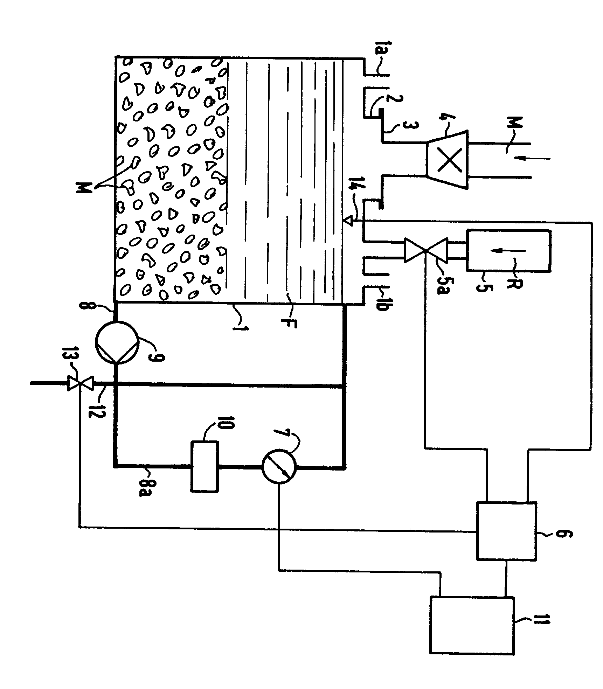

Das Verfahren und die Einrichtung gemäß der Erfindung werden anhand der Zeichnung näher erläutert.

Die Zeichnung zeigt eine Einrichtung zur Prüfung der Auslaugbeständigkeit eines Materials.

Zur Prüfung eines Materials M, das insbesondere Schmelzgranulat aus einer Abfallbehandlungsanlage, z.B. aus einer Schwel-Brenn-Anlage ist, wird dieses in einen Behälter 1 eingebracht, in dem sich eine Flüssigkeit F, z.B. Wasser, befindet. Im Gasraum oberhalb des Flüssigkeitsspiegels kann sich ein Schutzgas, z.B. ein inertes Gas, befinden. Zum Durchspülen des Gasraumes kann dieser über einen Einlaßstutzen la und einen Auslaßstutzen 1b mit Inertgas gespült werden. Der Behälter 1 weist zum Einbringen des Materials M und auch zum Einfüllen der Flüssigkeit F einen Einfüllstutzen 2 auf, der durch einen Deckel 3 dicht verschlossen werden kann. Das Material M kann über eine Zerkleinerungsvorrichtung 4 in den Behälter 1 eingebracht werden. Nach dem Einbringen des Materials M wird in der Flüssigkeit F mindestens eine erste Zustandsgröße stetig, sprunghaft oder periodisch geändert. Dazu wird mindestens ein geeignetes Reagenz R über mindestens einen Reagenzbehälter 5 mit Dosiervorrichtung 5a in den Behälter 1 eingebracht. Die Dosiervorrichtung 5a ist elektrisch mit einer Steuereinheit 6 verbunden. Die zu ändernde erste Zustandsgröße kann der pH-Wert, das Redoxpotential, die elektrische Leitfähigkeit oder die Konzentration eines gelösten Stoffes sein. Von der Zustandsgröße hängt es ab, welches Reagenz R in den Behälter 1 eingespeist wird. Während die erste Zustandsgröße geändert wird, wird eine zweite Zustandsgröße, die eine der zuvor genannten Zustandsgrößen ist, kontinuierlich gemessen. Dabei können die erste und die zweite Zustandsgröße die gleiche Zustandsgröße sein, wenn z.B. die zeitliche Verzögerung, mit der sich diese Zustandsgröße einstellt, beachtet werden soll. Sie können auch unterschiedliche Zustandsgrößen sein. Mit der Flüssigkeit F im Behälter 1 steht mindestens ein Meßgerät 7 in Verbindung. Ein Meßgerät 7 kann in einem Kreislauf 8 für die Flüssigkeit F angeordnet sein. Es kann auch direkt im Behälter 1 oder in einem Bypaß 8a des Kreislaufes 8 angeordnet sein. Im Kreislauf 8 befindet sich eine Pumpe 9. Außerdem kann vor einem Meßgerät 7 ein Filter 10 zum Zurückhalten fester Teile angeordnet sein. Ein Meßgerät 7 ist wie auch die Steuereinheit 6 mit einer Auswerteeinheit 11 verbunden. Dort wird für einen bestimmten, vorgegebenen Verlauf der ersten Zustandsgröße der Verlauf der zweiten Zustandsgröße registriert. In der Auswerteeinheit 11 ist eine Kalibrierverlauf abgespeichert. Dieser Kalibrierverlauf wird gewonnen, indem der gleiche Meßvorgang durchgeführt wird, wenn kein Material M im Behälter 1 vorhanden ist. In der Auswerteeinheit 11 wird die Differenz aus dem Prüfverlauf und dem Kalibrierverlauf gebildet. Wenn die Differenz nicht gleichbleibend Null ist, muß ein Teil des Materials M während der Prüfung in der Flüssigkeit F gelöst worden sein. Es ist also keine Auslaugbeständigkeit des Materials M gegeben. Das Prüfverfahren nach der Erfindung ist mit der gezeigten Einrichtung so schnell durchführbar, daß eine fortlaufende Prüfung von Material M, das ständig nachgeliefert wird, möglich ist. Sollte die Flüssigkeitsmenge F durch die Einspeisung von Reagenz R zu groß werden, kann Flüssigkeit F über einen sonst verschlossenen Auslaß 12 abgeleitet werden. Im Auslaß 12 kann dazu eine Armatur 13 angeordnet sein, die zur Steuerung der Flüssigkeitsableitung mit der Steuereinheit 6 verbunden ist. Im Behälter 1 kann ein Niveaufühler 14 zum Erkennen des Flüssigkeitsniveaus im Behälter 1 angeordnet sein. Der Niveaufühler 14 ist mit der Steuereinheit 6 verbunden.

Das geschilderte Verfahren kann nach einem vollständigen Entleeren des Behälters 1 mit einer neuen Materialprobe innerhalb von kurzer Zeit, z.B. innerhalb weniger Stunden, wiederholt werden. Eine Änderung der Auslaugbeständigkeit ist folglich schnell zu erkennen. Dadurch können schnell Gegenmaßnahmen eingeleitet werden.

## Patentansprüche

1. Verfahren zur Prüfung eines Materials (M), insbesondere von Schmelzgranulat aus einer Abfallbehandlungsanlage, wobei das Material (M) in eine Flüssigkeit (F) eingebracht und seine Beständigkeit gegen Auslaugung geprüft wird,
**dadurch gekennzeichnet,** daß in der Flüssigkeit (F) nach dem Einbringen des Materials (M) mindestens eine Zustandsgröße stetig, sprunghaft oder periodisch geändert und dabei der Verlauf mindestens einer Zustandsgröße als Prüfverlauf registriert wird, daß zuvor in einer gleichen Flüssigkeit (F) ohne Materialeinbringung und sonst unter gleichen Bedingungen in gleicher Weise die gleiche mindestens eine Zustandsgröße geändert wird und der Verlauf der für den Prüfverlauf vorgesehenen Zustandsgröße als Kalibrierverlauf registriert wird und daß die Differenz von Prüfverlauf und Kalibrierverlauf gebildet wird, wobei die Differenz ein Maß für die Beständigkeit des Materials (M) gegen Auslaugung ist.

2. Verfahren nach Anspruch 1,
**dadurch gekennzeichnet,** daß mindestens eine Zustandsgröße aus der Gruppe pH-Wert, Redoxpotential, elektrische Leitfähigkeit und Konzentration eines gelösten Stoffes geändert und dabei der Verlauf einer Zustandsgröße aus dieser Gruppe registriert wird.

3. Verfahren nach einem der Ansprüche 1 oder 2,
**dadurch gekennzeichnet,** daß zur Änderung einer Zustandsgröße in die Flüssigkeit ein oder mehrere Reagenzien dosiert werden.

4. Verfahren nach einem der Ansprüche 1 bis 3,
**dadurch gekennzeichnet,** daß die Änderung einer Zustandsgröße automatisch gesteuert wird.

5. Verfahren nach einem der Ansprüche 1 bis 4,
**dadurch gekenzeichnet,** daß das Material (M) vorzerkleinert in die Flüssigkeit (F) eingebracht wird.

6. Verfahren nach einem der Ansprüche 1 bis 5,
**dadurch gekennzeichnet,** daß das Material (M) von der Flüssigkeit (F) umspült wird.

7. Verfahren nach Anspruch 6,
**dadurch gekennzeichnet,** daß die Flüssigkeit (F) in einem geschlossenen Kreislauf (8) umläuft.

8. Verfahren nach Anspruch 7,
**dadurch gekennzeichnet,** daß die Flüssigkeit (F) im geschlossenen Kreislauf (8) unter einem gegenüber dem Außendruck erhöhten Druck umläuft.

9. Verfahren nach einem der Ansprüche 7 oder 8,
**dadurch gekennzeichnet,** daß die Temperatur der Flüssigkeit (F) im geschlossenen Kreislauf (8) gegenüber der Außentemperatur geändert wird.

10. Verfahren nach einem der Ansprüche 1 bis 9,
**dadurch gekennzeichnet**, daß ein Gasraum oberhalb der Flüssigkeit (F) mit einem Schutzgas gefüllt ist.

11. Verfahren nach einem der Ansprüche 7 bis 10,
**dadurch gekennzeichnet,** daß der Verlauf einer Zustandsgröße in einem abgezweigten Teilstrom der Flüssigkeit (F) registriert wird.

12. Verfahren nach einem der Ansprüche 1 bis 11,
**dadurch gekennzeichnet,** daß der Verlauf einer Zustandsgröße hinter einem Filter (10) registriert wird.

13. Verfahren nach einem der Ansprüche 1 bis 12,
**dadurch gekennzeichnet,** daß ein Teil der Flüssigkeit (F) entfernt und verworfen wird.

14. Einrichtung zur Prüfung eines Materials (M) auf Auslaugbeständigkeit, insbesondere von Schmelzgranulat aus einer Abfallbehandlungsanlage, mit einem Behälter (1) zur Aufnahme einer Flüssigkeit (F) und des Materials (M),
**dadurch gekennzeichnet,** daß der Behälter (1) eine Öffnung aufweist zum Hineingeben eines Reagenz (R), durch das eine Zustandsgröße der Flüssigkeit (F) geändert wird, daß dem Behälter (1) mindestens ein Meßgerät (7) zugeordnet ist zum Messen und Registrieren eines Prüfverlaufes der Zustandsgröße, daß das mindestens eine Meßgerät (7) mit einer Auswerteeinheit (11) zum Bilden der Differenz aus dem Prüfverlauf und einem Kalibrierverlauf verbunden ist, und daß der Behälter (1) in einem Kreislauf (8) für die Flüssigkeit (F), in dem sich eine Pumpe (9) befindet, angeordnet ist.

15. Einrichtung nach Anspruch 14,
**dadurch gekennzeichnet,** daß der Öffnung zum Hineingeben des Reagenz (R) mindestens eine Dosiervorrichtung (5a) für das Reagenz (R) vorgeschaltet ist.

16. Einrichtung nach Anspruch 15,
**dadurch gekennzeichnet,** daß die Dosiervorrichtung (5a) mit einer Steuereinheit (6) verbunden ist.

17. Einrichtung nach einem der Ansprüche 14 bis 16,
**dadurch gekennzeichnet,** daß dem Behälter (1) eine Zerkleinerungsvorrichtung (4) für das Material (M) vorgeschaltet ist.

18. Einrichtung nach einem der Ansprüche, 14 bis 17,
**dadurch gekennzeichnet,** daß des mindestens eine Meßgerät (7) im Kreislauf (8) außerhalb des Behälters (1) angeordnet ist.

19. Einrichtung nach Anspruch 18,
**dadurch gekennzeichnet,** daß das mindestens eine Meßgerät (7) in einem Bypaß (8a) des Kreislaufes (8) angeordnet ist.

20. Einrichtung nach einem der Ansprüche 18 oder 19,
**dadurch gekennzeichnet,** daß dem Meßgerät (7) ein Filter (10) vorgeschaltet ist.

21. Einrichtung nach einem der Ansprüche 14 bis 20,
**dadurch gekennzeichnet,** daß der Behälter (1) einen Auslaß (12) für überschüssige Flüssigkeit (F) aufweist.

22. Einrichtung nach einem der Ansprüche 14 bis 21,
**dadurch gekennzeichnet,** daß der Behälter (1) bis auf Anschlüsse geschlossen ist.

## Claims

1. Process for testing a material (M), preferably melt granules from a waste treatment plant, the material (M) being introduced into a liquid (F) and its resistance to leaching being tested, characterised in that following the introduction of the material (M), at least one variable is continuously, discontinuously or periodically changed in the liquid (F) and during this the course of at least one variable is recorded as the test run, in that previously, without introduction of material and otherwise under identical conditions in an identical manner, the at least one identical variable is changed in an identical liquid (F) and the course of the variable planned for the test run is recorded as the calibration run and in that the difference between test run and calibration run is formed, the difference being a measure of the resistance of the material (M) to leaching.

2. Process according to Claim 1, characterised in that at least one variable selected from the group comprising pH, redox potential, electric conductivity and concentration of a dissolved substance is changed and during this the course of a variable from this group is recorded.

3. Process according to either of Claims 1 and 2, characterised in that one or more reagents are added to change a variable in the liquid.

4. Process according to one of Claims 1 to 3, characterised in that the change in a variable is automatically controlled.

5. Process according to one of Claims 1 to 4, characterised in that the material (M) is introduced into the liquid (F) in a precomminuted state.

6. Process according to one of Claims 1 to 5, characterised in that the liquid (F) flows round the material (M).

7. Process according to Claim 6, characterised in that the liquid (F) is circulated in a closed circulation (8).

8. Process according to Claim 7, characterised in that the liquid (F) circulates in the closed circulation (8) at a pressure elevated with respect to the external pressure.

9. Process according to either of Claims 7 and 8, characterised in that the temperature of the liquid (F) in the closed circulation (8) is changed with respect to the external temperature.

10. Process according to one of Claims 1 to 9, characterised in that a gas space above the liquid (F) is filled with a protective gas.

11. Process according to one of Claims 7 to 10, characterised in that the course of a variable is recorded in a branched-off part-stream of the liquid (F).

12. Process according to one of Claims 1 to 11, characterised in that the course of a variable is recorded downstream of a filter (10).

13. Process according to one of Claims 1 to 12, characterised in that a part of the liquid (F) is removed and discarded.

14. Device for testing a material (M) for leaching resistance, preferably melt granules from a waste treatment plant, having a vessel (1) for receiving a liquid (F) and the material (M), characterised in that the vessel (1) has an opening for addition of a reagent (R) by means of which a variable is changed in the liquid (F), in that at least one measuring device (7) is assigned to the vessel (1) for measuring and recording the variable of a test run, in that the at least one measuring device (7) is connected to an evaluation unit (11) for forming the difference between the test run and a calibration run, and in that the vessel (1) is arranged in a circulation (8) for the liquid (F), in which circulation a pump (9) is located.

15. Device according to Claim 14, characterised in that at least one metering instrument (5a) for the reagent (R) is connected upstream of the opening for addition of the reagent (R).

16. Device according to Claim 15, characterised in that the metering instrument (5a) is connected to a control unit (6).

17. Device according to one of Claims 14 to 16, characterised in that a comminution instrument (4) for the material (M) is connected upstream of the vessel (1).

18. Device according to one of Claims 14 to 17, characterised in that the at least one measuring device (7) is arranged in the circulation (8) outside the vessel (1).

19. Device according to Claim 18, characterised in that the at least one measuring instrument (7) is arranged in a by-pass (8a) of the circulation (8).

20. Device according to either of Claims 18 and 19, characterised in that a filter (10) is connected upstream of the measuring instrument (7).

21. Device according to one of Claims 14 to 20, characterised in that the vessel (1) is equipped with an outlet (12) for excess liquid (F).

22. Device according to one of Claims 14 to 21, characterised in that the vessel (1) is sealed except for connections.

## Revendications

1. Procédé de test d'un matériau (M), notamment de granulés fondus provenant d'une installation de traitement de déchets, qui consiste à introduire le matériau (M) dans un liquide (F) et à tester sa résistance à la lixiviation, caractérisé en ce qu'il consiste à modifier dans le liquide (F) après l'introduction du matériau (M), constamment, par à-coups ou périodiquement, au moins une variable d'état et à enregistrer la courbe de cette au moins une valeur d'état comme courbe de test, à modifier auparavant dans un même liquide (F) sans introduction du matériau et sinon dans les mêmes conditions, de la même façon, la même au moins une variable d'état et à enregistrer la courbe de la variable d'état prévue pour le déroulement du test en tant que courbe de calibrage et à former la différence de la courbe de test et de la courbe de calibrage, la différence représentant une mesure de la résistance du matériau (M) à la lixiviation.

2. Procédé suivant la revendication 1,
caractérisé en ce qu'il consiste à modifier au moins une variable d'état choisie dans le groupe du pH, du potentiel rédox, de la conductivité électrique et de la concentration d'une substance dissoute et à enregistrer la courbe d'une variable d'état de ce groupe.

3. Procédé suivant l'une des revendications 1 ou 2,
caractérisé en ce qu'il consiste, pour modifier une variable d'état, à ajouter de manière dosée au liquide un réactif ou plusieurs réactifs.

4. Procédé suivant l'une des revendications 1 à 3,
caractérisé en ce qu'il consiste à commander automatiquement la modification d'une variable d'état.

5. Procédé suivant l'une des revendications 1 à 4,
caractérisé en ce qu'il consiste à introduire le matériau (M) dans le liquide (F) en l'ayant fragmenté à l'avance.

6. Procédé suivant l'une des revendications 1 à 5,
caractérisé en ce qu'il consiste à faire passer le liquide (F) sur le matériau (M).

7. Procédé suivant la revendication 6,
caractérisé en ce qu'il consiste à faire circuler le liquide (F) en circuit (8) fermé.

8. Procédé suivant la revendication 7,
caractérisé en ce qu'il consiste à faire passer le liquide en circuit (8) fermé sous une pression plus grande que la pression extérieure.

9. Procédé suivant l'une des revendications 7 ou 8,
caractérisé en ce qu'il consiste à modifier la température du liquide (F) dans le circuit (8) fermé par rapport à la température extérieure.

10. Procédé suivant l'une des revendications 1 à 9,
caractérisé en ce qu'il consiste à remplir un espace réservé au gaz au-dessus du liquide (F) d'un gaz protecteur.

11. Procédé suivant l'une des revendications 7 à 10,
caractérisé en ce qu'il consiste à enregistrer la courbe d'une variable d'état dans un courant partiel en dérivation du liquide (F).

12. Procédé suivant l'une des revendications 1 à 11,
caractérisé en ce qu'il consiste à enregistrer la courbe d'une variable d'état en aval d'un filtre (10).

13. Procédé suivant l'une des revendications 1 à 12,
caractérisé en ce qu'il consiste à éliminer une partie du liquide (F) et à la jeter.

14. Dispositif de test de la résistance à la lixiviation d'un matériau (M), notamment de granulés fondus provenant d'une installation de traitement des déchets, comprenant une cuve (1) de réception d'un liquide (F) et du matériau (M),
caractérisé en ce que la cuve (1) comporte une ouverture destinée à l'introduction d'un réactif (R) par lequel une variable d'état du liquide (F) est modifiée, en ce qu'à la cuve (1) est associé au moins un appareil (7) de mesure destiné à mesurer et à enregistrer une courbe de test de la variable d'état, en ce que ledit au moins appareil (7) de mesure est relié à une unité (11) de traitement destinée à former la différence entre la courbe de test et une courbe de calibrage, et en ce que la cuve (1) est disposée dans un circuit (8) pour le liquide (F), circuit dans lequel se trouve une pompe (9).

15. Dispositif suivant la revendication 14,
caractérisé en ce qu'en amont de l'ouverture d'introduction du réactif (R) est monté au moins un dispositif (5a) d'addition dosée du réactif (R).

16. Dispositif suivant la revendication 15,
caractérisé en ce que le dispositif (5a) d'addition dosée est relié à une unité (6) de commande.

17. Dispositif suivant l'une des revendications 14 à 16,
caractérisé en ce qu'en amont de la cuve (1) est monté un dispositif (4) de fragmentation du matériau (M).

18. Dispositif suivant l'une des revendications 14 à 17,
caractérisé en ce que le au moins un appareil (7) de mesure est monté dans le circuit (8) à l'extérieur de la cuve (1).

19. Dispositif suivant la revendication 18,
caractérisé en ce qu'au moins un appareil (7) de mesure est monté dans une dérivation (8a) du circuit (8).

20. Dispositif suivant l'une des revendications 18 ou 19,
caractérisé en ce qu'en amont de l'appareil (7) de mesure est monté un filtre (10).

21. Dispositif suivant l'une des revendications 14 à 20,
caractérisé en ce que la cuve (1) comporte une sortie (12) pour du liquide (F) en excès.

22. Dispositif suivant l'une des revendications 14 à 21,
caractérisé en ce que la cuve (1) est fermée à l'exception de raccordements.
